(19) **Europäisches Patentamt European Patent Office Office européen des brevets**

(11) **EP 4 066 649 B1**

(12) **EUROPEAN PATENT SPECIFICATION**

(45) Date of publication and mention
of the grant of the patent:
**02.07.2025 Bulletin 2025/27**

(21) Application number: **22165130.0**

(22) Date of filing: **29.03.2022**

(51) International Patent Classification (IPC):
*A23K 20/20* (2016.01)    *A23K 50/30* (2016.01)
*A23K 50/60* (2016.01)    *A61K 33/30* (2006.01)
*A61P 1/12* (2006.01)    *C01G 9/00* (2006.01)

(52) Cooperative Patent Classification (CPC):
**A23K 20/30; A23K 50/30; A23K 50/60;
A61K 33/30; A61P 1/12**

(54) **SINGLE ATOMIC ZINC ADDITIVE FOR USE IN FEEDS AND PREPARATION METHOD THEREOF**

EINATOMIGER ZINKZUSATZ ZUR VERWENDUNG IN FUTTERMITTELN UND
HERSTELLUNGSVERFAHREN DAVON

ADDITIF ATOMIQUE UNIQUE DE ZINC DESTINÉ À ÊTRE UTILISÉ DANS DES ALIMENTS ET SON
PROCÉDÉ DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.04.2021 CN 202110353134**

(43) Date of publication of application:
**05.10.2022 Bulletin 2022/40**

(73) Proprietor: **Linkway Technology Co., Ltd.
Nanning Pilot Free Trade Zone, Nanning Area
Guangxi
530028 (CN)**

(72) Inventors:
• **ZHAO, Chao
Nanning Pilot Free Trade Zone (Nanning Area),
Guangxi, 530028 (CN)**

• **WANG, Jing
Nanning Pilot Free Trade Zone (Nanning Area),
Guangxi, 530028 (CN)**
• **HUANG, Hongfeng
Nanning Pilot Free Trade Zone (Nanning Area),
Guangxi, 530028 (CN)**
• **WU, Yubo
Nanning Pilot Free Trade Zone (Nanning Area),
Guangxi, 530028 (CN)**

(74) Representative: **Patent 42
5, rue Dicks
P.O.Box BP297
4081 Esch-zur-Alzette (LU)**

(56) References cited:
WO-A1-03/077674      CN-A- 1 327 739
CN-A- 109 349 431    CN-A- 109 482 230
CN-A- 111 266 099    CN-A- 112 451 734
CN-B- 106 186 042

Description

## TECHNICAL FIELD

The present application relates to the technical field of animal feed additives, and particularly to a single atomic zinc additive for replacing nano zinc oxide for use in feeds and a preparation method thereof.

## BACKGROUND

At present, due to an overall consideration on sow reproduction and production efficiency in pig industry, piglets aged 3-4 weeks will be generally weaned, which, however, often leads to diarrhea in piglets weaned at this early stage.

It has been found that the nano zinc oxide with a concentration of 1500-3000 ppm can reduce the occurrence of diarrhea in piglets. The nano zinc oxide can inhibit the activation and proliferation of intestinal mast cells, improve autoimmune response in piglets, and prevent the reproducing of conditional pathogens, for example, Escherichia coli and Enterococcus, in the intestine. Therefore, at present, the most effective way to prevent and control diarrhea in weaned piglets is to use a high-dose zinc (with a zinc oxide content of about 2500 ppm).

However, with the increased use of the high-dose zinc, it has been found that, high-dose of zinc leads to a negative influence. That is, excessive zinc, since it cannot be absorbed by piglets, is excreted out of the body of piglets with its feces. The feces containing zinc may enter the soil to pollute the local environment. Long-term use of the high-dose zinc will lead to a negative influence on the piglets, and even lead to tolerance to some drugs in the piglets, which will increase the cost of drugs. Most of the high-dose zinc will be neutralized into $Zn^{2+}$ by gastric acid, but a large amount of $Zn^{2+}$ will negatively affect the absorption of trace elements such as copper, iron and selenium in piglets.

CN106186042B discloses a preparation method of three-dimensional hierarchical porous nano zinc oxide for feed, which includes the following steps: preparing 0.2-0.4 mol/l of zinc salt solution, and adding 50-150 g of diatomite into each liter of the zinc salt solution to mix into suspension; preparing 0.2-0.4 mol/l of ammonia water/carbonate solution, slowly dripping 1-1.2 times of the zinc salt solution in the step (1) into the suspension in the step (1) while stirring until the reaction is complete; and (3) carrying out centrifugal separation on the solution obtained in the step (2), washing and drying the obtained precipitate by using deionized water, and calcining the precipitate at the high temperature of 300-600 °C for 2-4 hours to obtain a product, namely the three-dimensional hierarchical porous nano zinc oxide for the feed. The three-dimensional hierarchical porous nano zinc oxide for the feed has larger specific surface area, more effective sterilization effect and less environmental pollution, and can better prevent the diarrhea of weaned pigs caused by stress. CN109349431A discloses a preparation method of a diarrhea-preventing suckling pig feed additive, and the method includes the following steps of (1) surface modification of palygorskite: gradient heating the palygorskite, and then modifying the palygorskite by using a high-efficiency active dispersant to obtain modified palygorskite; (2) compounding and cementing zinc oxide and palygorskite: mixing the modified palygorskite powder with the composite zinc oxide, then adding an adhesion promoter solution to perform functional compound cementation on the modified palygorskite and the zinc oxide, magnetically stirring uniformly, drying and grinding into powder to obtain the additive. In a word, the piglet feed additive prepared by the invention has a high-efficiency diarrhea prevention effect, can improve the gastrointestinal environment of piglets, and improves the gastrointestinal adaptability of piglets in the weaning period. WO2003077674A1 discloses a feed additive of nanometer size and a process for preparing the same. Said additive consists of a composition of copper modified montmorillonite and zinc modified montmorillonite in a weight proportion of 1:0.5-20. Said additive has such extreme high antibiosis and antiviral properties that it can be used to substitute for the antibiotics and growth promotion feed additives. The present invention also relates to a feed containing the feed additive of nanometer size.

## SUMMARY

In order to reduce the use amount of zinc and improve the effect of zinc on preventing diarrhea in piglets, promote the growth of piglets and reduce the pollution of residual zinc on the environment, the present application provides a single atomic zinc additive for replacing nano zinc oxide for use in feeds and a preparation method thereof.

In a first aspect, the present application provides a single atomic zinc additive for replacing nano zinc oxide for use in feeds.

A single atomic zinc additive for use in feeds includes a carrier and active metal zinc. The active metal zinc is distributed on the surface of the carrier in single atomic form, without active metal zinc contained inside the carrier.

In an animal, only zinc atoms in direct contact with the animals can work. However, in a conventional process, most of the zinc in nano zinc oxide is wrapped inside, having a low use rate of zinc atoms. In the present applicant, the active metal zincs are distributed on the surface of the carrier in single atomic form, and there is no active metal zinc contained inside the carriers, so that the utilization rate of the zinc atom can be improved. In the bodies of animals, zinc oxide will be neutralized into $Zn^{2+}$ by gastric acid, but a large amount of $Zn^{2+}$ will affect the absorption of trace elements such as copper, iron and selenium in piglets. In the present applicant, single atomic zincs are loaded on the surface of carriers, there will be no residual $Zn^{2+}$ in the present application since zinc will not be changed into $Zn^{2+}$ due to the carrying effect of the carriers. Therefore, zinc can achieve the effect of treating diarrhea, promote the growth of piglets and cause no pollution on the environment in the present application.

Further, the carrier is nano silica, and a mass ratio of metal zinc to nano silica is 1:(100-200).

By controlling the mass ratio of the active metallic and the carrier to control the amount of metal loaded on the carrier, the active metal loaded on each carrier can play the best effect.

In a second aspect, the present applicant provides a preparation method of single atomic zinc additive for use in feeds, including the following steps:

S1: preparation of a nano silica carrier;

S2: preparation of a single atomic precursor of active metal zinc;

S3: preparation of single atomic zinc additive precursor: adding the nano silica carrier obtained in S1 to a mixture solution obtained in S2 at a speed of 30-60 g/min, performing ultrasonic treatment for 0.2-1 h, stirring at 450-550 rpm for 10-20 h, washing with water to neutral, filtering, drying and grinding to 500 nm to obtain the single atomic zinc additive precursor powder; and

S4: activation of the single atomic zinc additive precursor powder obtained in S3 at a high temperature to obtain a final product.

By ultrasonic treatment of the carrier and the single atomic precursors of active metals, the active metal can be more evenly distributed on the surface of the carrier, thereby improving the effect of treating piglet diarrhea and promoting the growth of piglets by the present method.

Further, the preparation method of nano silica carrier includes the following steps:

S11: preparing a sodium silicate solution;

S12: stirring an aqueous ammonium chloride solution by 400-600 rpm at 35-45 °C, and dripping 0.3-0.5 mol/L sodium silicate solution into the aqueous ammonium chloride solution by 5-15 μL/s until pH is 8.5;

S13: stirring the solution obtained in S12 by 400-600 rpm for 0.5-1.5 h at 35-45 °C to obtain a precipitate, and centrifugally washing the precipitate with a washing solution; and

S14: drying the precipitate obtained in S13 at 90-110 °C, and then calcining at 480-520 °C for 0.5-1.5 h to obtain the nano silica carrier.

Dripping the sodium silicate solution into aqueous ammonium chloride solution can control the reaction process. Washing the mixed liquid prepared by mixing and stirring can further improve the dispersion degree of silica, reduce the possibility of silica agglomeration, improve the bonding strength between the carrier and zinc atoms, improve the effect of the product prepared in the present application on diarrhea, and promote the growth of piglets, causing no pollution to the environmental.

Further, in S11, the sodium silicate solution is prepared by the following steps: preparing an aqueous ethanol solution by a volume ratio of ethanol to water of 1: (7-9), adding a sodium silicate solution to the aqueous ethanol solution to obtain a mixed solution of 0.3-0.5 mol/L, and adding cationic surfactant cetyltrimethylammonium bromide (CTAB) to the mixed solution to obtain the sodium silicate solution.

Cationic surfactant cetyltrimethylammonium bromide is a cationic surfactant with excellent emulsifying effect, which can provide a well-mixed aqueous ethanol solution and sodium silicate solution. Cetyltrimethylammonium bromide can reduce the agglomeration of nano silica and improve the uniformity and stability of the obtained sodium silicate solution. Cetyltrimethylammonium bromide has biodegradability and sterilizing function itself, so that the sterilizing effect of the present application can be further improved.

Further, 0.3-0.7 g cationic surfactant cetyltrimethylammonium bromide is added per 100 mL of the sodium silicate solution.

By controlling the use amount of cetyltrimethylammonium bromide, the possibility of precipitation due to a high concentration can be reduced, so that cetyltrimethylammonium bromide can improve the stability of sodium silicate solution while giving full play to its efficacy.

Further, the washing solution in S13 is obtained by mixing cetyltrimethylammonium bromide with the aqueous ethanol solution, in which the volume ratio of ethanol to water in aqueous ethanol solution is 1: (7-9), and 0.8-1.2 mol of cetyltrimethylammonium bromide is contained per 100 mL of the aqueous ethanol solution.

Cetyltrimethylammonium bromide is easily soluble in ethanol. Adding cetyltrimethylammonium bromide to the ethanol solution can further reduce the agglomeration of nano silica, so that more zinc atoms can be loaded on the surface of the carrier to improve the utilization rate of the carrier.

Further, in S2, the single atomic precursor of active metal zinc is prepared by the following steps: adding the sodium carbonate solution to zinc nitrate solution by 5-15 μL/s, stirring and mixing at 400-600 rpm for 2-4 h, heating to 55-70 °C within 20-40 min, performing mixing and stirring at the same speed for another 2-4 h, and then cooling to room temperature to obtain the single atomic precursor of active metal zinc.

By adding sodium carbonate into the zinc nitrate solution, the temperature of zinc nitrate solution is improved, and in turn the stability of the obtained mixture is improved.

Further, the sodium carbonate solution has a mass concentration of 5%, the zinc nitrate solution has a concentration of 100-200 g/L, and a volume ratio of the sodium carbonate solution to the zinc nitrate solution is (20-40): 100.

By mixing sodium carbonate with zinc nitrate in proportion, the stability of the prepared mixed solution is further improved.

Further, in S4, the activation at a high temperature includes: activating the single atomic zinc additive precursor powder obtained in step S3 at a high temperature of 670-740 °C for 1.5-2.5 h in 5% hydrogen-argon mixture atmosphere, cooling, and grinding to 500 nm to obtain the single atomic zinc additive.

The hydrogen-argon mixture can reduce the metal single atomic precursor, avoid oxidation thereof at high temperature, and improve the ability of the atomic zinc additive prepared in the present application to activate oxygen, thereby improving the effect of the single atomic zinc additive prepared in the present application on the prevention of piglet diarrhea.

In summary, the present application achieves the following beneficial effects:

1. a small amount of single atomic zinc additive has the same effect of preventing piglet diarrhea and promoting piglet growth as a large amount of nano zinc oxide while solving the problem of zinc oxide increasing microbial drug resistance;

2. the content of metal element zinc in single atomic zinc additive is low and zinc can be completely absorbed by the body of animals, which solves the problem of environmental pollution caused by high-dose zinc; and

3. the preparation method according to the present applicant involves in readily available raw materials and simple operation.

**BRIEF DESCRIPTION OF THE DRAWINGS**

FIG. 1 is an effect diagram showing the distribution of zinc atoms in Example 3; and

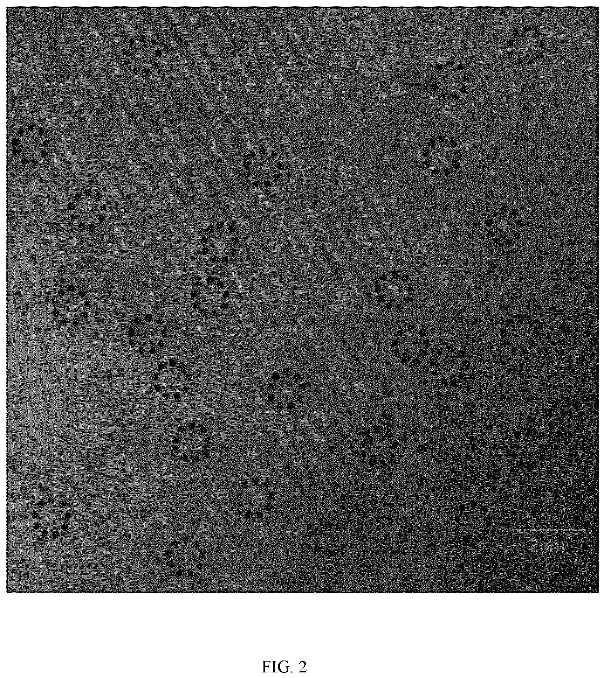

FIG. 2 is a scanning electron microscope diagram in Example 3.

**DETAILED DESCRIPTION**

The present application will be further described in details below in connection with the accompanying drawings.

**Preparation Examples**

Preparation Example 1

Preparation of washing solution: the washing solution was obtained by mixing cationic surfactant cetyltrimethylammonium bromide with an aqueous ethanol solution. The volume ratio of ethanol to water in the aqueous ethanol solution was 1:8, and 1 mol cetyltrimethylammonium bromide was contained per 100 mL of the aqueous ethanol solution.

**Example**

Example 1

A preparation method of single atomic zinc additive for replacing nano zinc oxide for use in feeds was performed by the following steps:

S1. preparation of a nano silica carrier, including the following steps:

S11: preparation of sodium silicate solution: sodium silicate and cationic surfactant cetyltrimethylammonium bromide were added to the aqueous ethanol solution prepared by a volume ratio of ethanol to water of 1:8, thereby obtaining a 0.4 mol/L sodium silicate solution, in which 0.5 g cetyltrimethylammonium bromide was contained per 100 mL solution;

S12: 100mL of 1.5 mol/L aqueous ammonium chloride solution was placed on a constant temperature magnetic stirrer, mixed and stirred by 500 rpm at 40 °C. The sodium silicate solution obtained in S11 was dripped by 10 μL/s into aqueous ammonium chloride solution until the pH of the mixture is 8.5;

S13: the mixture obtained in S12 was stirred by 500 rpm for 1 h at 40 °C to obtain a precipitate, and the precipitate was centrifuged and washed with the washing solution obtained in Preparation Example 1; and

S14: the precipitate obtained in S13 was dried at 100 °C in an oven and calcined at 500 °C in a muffle furnace for 1 h. Then, the precipitate was ground to 500 nm on a planetary mill (zirconia ball mill) to obtain a nano silica carrier.

S2: preparation of single atomic precursor of active metals: 20 mL of 5% aqueous sodium carbonate solution was slowly added into 100 mL of 100 g/L aqueous zinc nitrate solution at 10 μL/s, and then stirred at 500 rpm for 3 h. The mixture was heated to 60 °C within 30 min, stirred at constant speed for 3 h, and then cooled to room temperature to obtain a mixed solution.

S3. Preparation of single atomic zinc additive precursor: the carrier obtained in S1 was added to the mixed solution obtained in S2 at 50 g/min, in which the mass ratio of active metal zinc to nano silica carrier was 1:200. The mixed solution was ultrasonically treated for 0.5 h, stirred and mixed at 500 rpm for 12 h, thoroughly washed and filtered with water to neutral, dried, and ground to 500 nm on a planetary mill (zirconia ball mill) to obtain single atomic zinc additive precursor powder.

S4: high temperature activation treatment: the single atomic zinc additive precursor powder obtained in S3 was activated at a high temperature of 700 °C in a 5% hydrogen-argon mixture atmosphere for 2 h, cooled and ground to 500 nm on a planetary mill (zirconia ball mill) to obtain the single atomic zinc additive.

Example 2

A preparation method of single atomic zinc additive for replacing nano zinc oxide for use in feeds included the following steps:

S1. preparation of nano silica carrier, including the following steps:

S11: preparation of sodium silicate solution: sodium silicate and cationic surfactant cetyltrimethylammonium bromide were added to the aqueous ethanol solution prepared by a volume ratio of ethanol to water of 1:8, thereby obtaining a 0.4mol/L sodium silicate solution, in which 0.5 g cetyltrimethylammonium bromide was contained per 100 mL solution;

S12: 100 mL of 1.5 mol/L aqueous ammonium chloride solution was placed on a constant temperature magnetic stirrer, mixed and stirred by 500 rpm at 40 °C. The sodium silicate solution obtained in S11 was dripped by 10 μL/s into the aqueous ammonium chloride solution until the pH of the mixture was 8.5;

S13: the mixture obtained in S12 was stirred by 500 rpm for 1 h at 40 °C to obtain a precipitate, and the precipitate was centrifuged and washed with the washing solution obtained in Preparation Example 1; and

S14: the precipitate obtained in S13 was dried at 100 °C in an oven and calcined at 500 °C in a muffle furnace for 1 h. Then, the precipitate was ground to 500 nm on a planetary mill (zirconia ball mill) to obtain the nano silica carrier.

S2: preparation of single atomic precursor of active metals: 30 mL of 5% aqueous sodium carbonate solution was slowly added by 10 μL/s into 100 mL of 150 g/L aqueous zinc nitrate solution, and then stirred at 500 rpm for 3 h. The mixture was heated to 60 °C within 30 min, stirred at constant speed for 3 h, and then cooled to room temperature to obtain the mixture.

S3. preparation of single atomic zinc additive precursor: the carrier obtained in S1 was added to the mixed solution obtained in S2 at 50 g/min, in which the active metal zinc and the nano silica carrier had a mass ratio of 1:150. The mixed solution was ultrasonically treated for 0.5 h, stirred and mixed by 500 rpm for 12 h, thoroughly washed and filtered with water to neutral, dried and ground to 500 nm on a planetary mill (zirconia ball mill) to obtain the single atomic zinc additive precursor powder.

S4: high temperature activation treatment: the single atomic zinc additive precursor powder obtained in S3 was activated at a high temperature of 700 °C in a 5% hydrogen argon mixture atmosphere for 2 h, cooled, and ground to 500 nm on a planetary mill (zirconia ball mill) to obtain the single atomic zinc additive.

Example 3

A preparation method of single atomic zinc additive for replacing nano zinc oxide for use in feeds included the following steps:

S1. preparation of nano silica carrier, including the following steps:

S11: preparation of sodium silicate solution: sodium silicate and cationic surfactant cetyltrimethylammonium bromide were added to the aqueous ethanol solution by a volume ratio of ethanol to water of 1:8, thereby obtaining a 0.4 mol/L sodium silicate solution, in which 0.5 g cetyltrimethylammonium bromide was contained per 100 mL solution.

S12: 100 mL of 1.5 mol/L aqueous ammonium chloride solution was placed on a constant temperature magnetic stirrer, mixed and stirred under 500 rpm at 40 °C. The sodium silicate solution obtained in S11 was dripped by 10 μL/s into the aqueous ammonium chloride solution until the pH of the mixed solution was 8.5.

S13: the mixed solution obtained in S12 was stirred under 500 rpm for 1 h at 40 °C to obtain a precipitate, and the precipitate was centrifuged and washed with the washing solution obtained in Preparation Example 1.

S14: the precipitate obtained in S13 was dried at 100 °C in an oven and calcined at 500 °C in a muffle furnace for 1 h. Then, the precipitate was ground to 500 nm on a planetary mill (zirconia ball mill) to obtain the nano silica carrier.

S2: preparation of single atomic precursor of active metals: 40 mL of 5% aqueous sodium carbonate solution was slowly added into 100 mL of 200 g/L aqueous zinc nitrate solution at 10 μL/s, and then stirred under 500 rpm for 3 h. The mixed solution was heated to 60 °C within 30 min, stirred at a constant speed for 3 h, and then cooled to room temperature to obtain a mixed solution.

S3. preparation of single atomic zinc additive precursor: the carrier prepared in S1 was added to the mixed solution prepared in S2 by 50 g/min, in which the mass ratio of active metal zinc to nano silica carrier was 1:100. The mixed solution was ultrasonically treated for 0.5 h, stirred and mixed at 500 rpm for 12 h, thoroughly washed and filtered with water to neutral, dried and ground to 500 nm on a planetary mill (zirconia ball mill) to obtain single atomic zinc additive precursor powder.

S4: high temperature activation treatment: the single atomic zinc additive precursor powder obtained in S3 was activated at a high temperature of 700 °C in a 5% hydrogen-argon mixture atmosphere for 2 h, cooled and ground to 500 nm on a planetary mill (zirconia ball mill) to obtain a single atomic zinc additive.

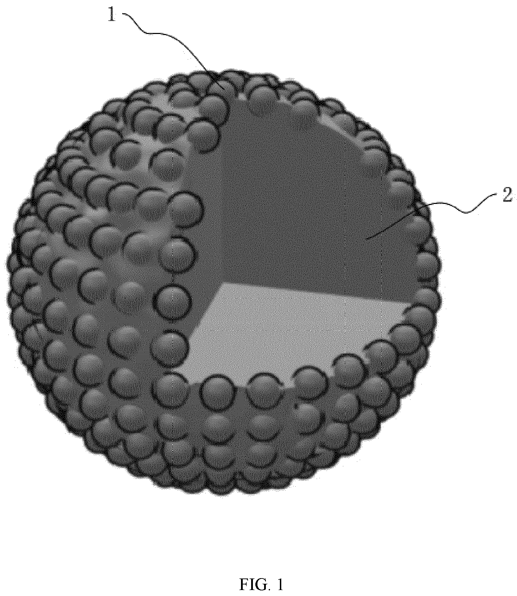

Referring to FIG.1, in the single atomic zinc additive for replacing nano zinc oxide for use in feeds in the present applicant, zinc atom 1 is ankled on the surface of the nano-silica carrier 2, and there is no zinc atom 1 contained inside the nano-silica carrier 2.

Comparative Example 1

Comparative Example 1 differs from Example 1 in that: for preparing single atomic zinc additive precursor in S3, in Comparative Example 1, the mass ratio of active metal zinc and nano silica carrier in S3 is 1:300.

Comparative Example 2

Comparative Example 2 differs from Example 1 in that: for preparing single atomic zinc additive precursor in S3, in Comparative Example 1, the mass ratio of active metal zinc and nano silica carrier in S3 is 1:80.

Example 4

Example 4 differs from Example 1 in the high-temperature for activating in S4. In particular, in S4 of Example 4, the single atomic zinc additive precursor powder prepared in S3 was activated at a high temperature of 700 °C in a 5% air atmosphere for 2 h, cooled and ground to 500 nm on a planetary mill (zirconia ball mill) to obtain the single atomic zinc additive.

Example 5

Example 5 differs from Example 1 in S13. In particular, in S13 of Example 5, the mixed solution obtained in S12 was continuously mixed and stirred under 500 rpm at 40 °C for 1 h to obtain a precipitate, which was centrifugally washed with water.

Example 6

Example 6 differs from Example 1 in the sodium silicate solution in S11. In particular, in S11 of Example 6, sodium silicate and cationic surfactant cetyltrimethylammonium bromide were added to the aqueous ethanol solution with a volume ratio of ethanol to water of 1:8 to obtain 0.4 mol/L sodium silicate solution, in which 0.7 g CTAB was contained per 100 mL solution.

Example 7

Example 7 differs from Example 1 in the sodium silicate solution in S11. In particular, in S11 of Example 7, sodium silicate and cationic surfactant cetyltrimethylammonium bromide were added to the aqueous ethanol solution with a volume ratio of ethanol to water of 1:8 to obtain 0.4 mol/L sodium silicate solution, in which 0.3 g cetyltrimethylammonium bromide was contained per 100 mL solution.

Example 8

Example 8 differs from Example 1 in the preparation method of the active metal single atomic precursor of S2. In particular, in S2 of Example 8, 20 mL 5% aqueous sodium carbonate solution was directly added into 100 mL of 100 g/L aqueous zinc nitrate solution, and then stirred under 500 rpm for 3 h. The mixed solution was heated to 60 °C within 30 min, stirred at a constant speed for 3 h, and then cooled to room temperature to obtain the mixed solution.

Example 9

Example 9 differs from Example 1 in the preparation method of the active metal single atomic precursor of S2. In particular, in S2 of Example 9, 20 mL 5% aqueous sodium carbonate solution was directly added into 100 mL of 100 g/L aqueous zinc nitrate solution at 10 $\mu$L/s, and then stirred at 500 rpm, 60 °C for 3 h, and then cooled to room temperature to obtain the mixed solution.

Example 10

Example 10 differs from Example 1 in the preparation method of single atomic zinc additive precursor in S3. In particular, in S2 of Example 10, the carrier prepared in S1 was added to the mixed solution obtained in S2 at 50 g/min. The mass ratio of active metal zinc to nano silica carrier was 1:200. The mixed solution was stirred and mixed at 500 rpm for 12 h, thoroughly washed and filtered with water to neutral, dried and ground to 500 nm on a planetary mill (zirconia ball mill) to obtain single atomic zinc additive precursor powder.

**Performance Test**

1. Toxicological test

1.1 Acute toxicity test (LD50):

According to the use amount in Examples 1-3, two dose groups of 2000 mg/kg and 3000 mg/kg and a blank control group were established for each type. 18-22 g white mice were divided into groups, 10 mice in each group, half male and half female. Two dose groups were used for Test A and Test B, respectively. The mice in the blank control group were not given drugs, and the mice in Test group A and Test group B were continuously given drugs with preset doses for 7 days. The spirit, appetite, water drinking, activity and poisoning of the mice were observed every day, and the death number of mice was recorded.

1.2 Cumulative toxicity test (20 days cumulative test method):

15-18 g white mice were divided into groups, 10 mice in each group, half male and half female. For the single atomic zinc additives prepared in Example 1-3, each was tested with two dose groups and one control group. The comparison was repeated and divided into Test group A and Test group B. The mice in the blank control group were not given drugs, and the mice in test groups were intragastrically administered by two doses, that is, 2000 mg/kg and 3000 mg/kg, for 20 days. During the administration period, the spirit, appetite, death and abnormal reactions of the mice were observed. After the administration was stopped, the death and weight changes of mice were observed within 7 days.

2. Piglet diarrhea test

Test scheme: weaned piglets were fed with zinc oxide feeds and single atomic zinc additives prepared in Examples 1-3, and the effects of single atomic zinc additive and zinc oxide on diarrhea and production performance of piglets were studied. A batch of 20 weaned piglets with diarrhea were observed. After weaned piglets were fed with normal pig food in the first two days, they were randomly divided into 4 groups with 5 pigs in each group. After 14 days, the piglets in the 4 groups were fed with 2000 ppm single atomic zinc additive and zinc oxide in addition to normal pig food. Piglets were kept indoors under the same temperature, humidity and ventilation conditions.

Index determination and method: the test period was 14 days, during which the feeding amount in each feeding was accurately recorded. The piglets and the remaining feed were weighed every week, and the average daily feed intake, average daily gain and feed conversion rate of piglets were measured accordingly. The mortality and the content of zinc in piglet feces were recorded every day (the content of zinc was measured by slurry method using a dissolution instrument).

3. Antimicrobial resistance test:

The single atom zinc additive and nano zinc oxide for use in feeds prepared in examples 1-4, and Comparative Examples 1-2 were tested for drug resistance antibacterial activity:

Step 1: bacteria (drug-resistant Escherichia coli, drug-resistant Clostridium welchii and Methicillin-resistant Staphylococcus aureus) freshly cultured for 18-24 h were prepared. The bacteria were washed to remove fungus moss and prepared into a bacterial suspension with 5 mL PBS solution (0.03 mol/L). Then the bacterial suspension was diluted with PBS solution to desired concentrations (100 $\mu$L bacterial suspension was dropped on a control sample tablet, and the number of recovered bacteria was $1 \times 10^4$-$9 \times 10^4$ cfu/tablet);

Step 2: a certain amount of single atomic zinc additive and zinc oxide were weighed and dispersed in PBS solution to form a sample solution (the concentration of single atomic zinc additive and zinc oxide was 2000 ppm), which was put into a 250 mL conical flask;

Step 3: the conical bottle was fixed on a shaking table and shaken at 300 r/min for 1 h; and

Step 4: 0.5 mL of the sample solution (or sample solution properly diluted with PBS) were taken at 0 h and after shaking for 1 h respectively, inoculated in a flat dish with agar pouring method, and, incubated in 36-37 °C incubator for 18-24 h, followed by bacterial colonies counting.

The test was repeated for 3 times, and the antibacterial rate was calculated according to the formula:

$$X = (A\text{-}B)/A \times 100\%$$

where,

X - antibacterial rate, %;
A - average number of colonies of the sample before shaking;
B - average number of colonies of the sample after shaking.

Detection method/Testing method

Table 2 Acute toxicity test

| Groups | Test group A | | | | Test group B | | | |
|---|---|---|---|---|---|---|---|---|
| Example 1 | Routine observation | Average weight gain | Death | LD50 | Routine observation | Average weight gain | Death | LD50 |
| gavage 2000mg/kg | normal | 13.1a±1. 6 | no | >2000 mg/kg | normal | 13.2a±1.4 | 无 | >2000 mg/kg |
| gavage 3000mg/kg | normal | 13.2a±1. 5 | no | >3000 mg/kg | normal | 13.3a±1.5 | no | >3000 mg/kg |
| blank control group | normal | 13.2a±1. 5 | no | | normal | 13.2a±1.6 | no | |
| Example 2 | Routine observation | Average weight gain | Death | LD50 | Routine observation | Average weight gain | Death | LD50 |
| gavage 2000mg/kg | normal | 13.2a±1. 6 | no | >2000 mg/kg | normal | 13.1a±1.5 | no | >2000 mg/kg |

(continued)

| Groups | Test group A | | | | Test group B | | | |
|---|---|---|---|---|---|---|---|---|
| Example 1 | Routine observation | Average weight gain | Death | LD50 | Routine observation | Average weight gain | Death | LD50 |
| gavage 3000mg/kg | normal | 13.2a±1.6 | no | >3000 mg/kg | normal | 13.1a±1.6 | no | >3000 mg/kg |
| blank control group | normal | 13.1a±1.5 | no | | normal | 13.2a±1.5 | no | |
| Example 3 | Routine observation | Average weight gain | Death | LD50 | Routine observation | Average weight gain | Death | LD50 |
| gavage 2000mg/kg | normal | 13.2a±1.6 | no | >2000 mg/kg | normal | 13.2a±1.4 | no | >2000 mg/kg |
| gavage 3000mg/kg | normal | 13.3a±1.3 | no | >3000 mg/kg | normal | 13.3a±1.5 | no | >3000 mg/kg |
| blank control group | normal | 13.2a±1.6 | no | | normal | 13.2a±1.4 | no | |

In the acute toxicity test, the mice in Test group A, Test group B and blank control group in Examples 1-3 showed no abnormal performance compared with that in the control group after the acute toxicity test 7 days, and the mice in the two test groups showed normal appetite, mental state, water drinking and food intake after the acute toxicity test. After administration of 3000 mg/kg single atomic feed additive in group A and group B respectively, no poisoning death occurred in the two groups of mice after acute toxicity test. According to the evaluation standard of toxicology, there was no need to determine the median lethal dose, that was, LD > 3000 mg/kg. The results of acute toxicity test showed that the single atomic zinc additive was non-toxic.

Table 3 Cumulative toxicity test

| Groups | Test group A | | | Test group B | | |
|---|---|---|---|---|---|---|
| Example 1 | Initial weight | End weight | Average weight gain | Initial weight | End weight | Average weight gain |
| gavage 5000mg/kg | 16.2a±1.5 | 29.3a±2.4 | 13.1a±1.6 | 16.3a±1.6 | 29.2a±2.6 | 13.2a±1.4 |
| gavage 10000mg/k g | 16.1a±1.6 | 29.4a±2.5 | 13.2a±1.5 | 16.4a±1.5 | 29.3a±2.6 | 13.3±1.5 |
| blank control group | 16.3a±1.5 | 29.2a±2.5 | 13.2a±1.5 | 16.5a±1.5 | 29.4a±2.5 | 13.2a±1.6 |
| Example 2 | Initial weight | End weight | Average weight gain | Initial weight | End weight | Average weight gain |
| gavage 5000mg/kg | 16.1a±1.5 | 29.4a±2.5 | 13.2a±1.6 | 16.5a±1.5 | 29.4±2.6 | 13.1a±1.5 |
| gavage 10000mg/k g | 16.3a±1.6 | 29.5a±2.5 | 13.2a±1.6 | 16.3a±1.6 | 29.5a±2.7 | 13.1a±1.6 |
| blank control group | 16.2a±1.5 | 29.3a±2.5 | 13.1a±1.5 | 16.4a±1.6 | 29.4a±2.7 | 13.2a±1.5 |
| Example 3 | Initial weight | End weight | Average weight gain | Initial weight | End weight | Average weight gain |
| gavage 5000mg/kg | 16.4a±1.7 | 29.3a±2.4 | 13.2a±1.6 | 16.4a±1.5 | 29.4a±2.7 | 13.2a±1.4 |
| gavage 10000mg/k g | 16.3a±1.5 | 29.4a±2.4 | 13.3a±1.3 | 16.5a±1.6 | 29.5a±2.4 | 13.3a±1.5 |
| blank control group | 16.3a±1.6 | 29.4a±2.6 | 13.2a±1.6 | 16.4a±1.4 | 29.5a±2.6 | 13.2a±1.4 |
| Note: the shoulder mark in the same column indicates no significant difference (P > 0.05) | | | | | | |

After the cumulative toxicity test, the mice in Test group A and Test group B in Examples 1-3 showed normal appetite, spirit and drinking water after administration 20 days, and there was no death. After stopping the Administration for 7 days, the mice showed normal performance. It can be seen from table 3 that after administration 20 days, there was no significant difference in initial weight, end weight and average weight gain between the mice in the two test groups using the single atomic zinc additive prepared in Examples 1-3 and the blank control group.

Table 4. Effects of single atomic zinc additive and nano zinc oxide for use in feeds prepared in Examples 1-3 on diarrhea and production performance of piglets

| Drugs | Example 1 | Example 2 | Example 3 | Nano zinc oxide for use in feeds |
|---|---|---|---|---|
| Content of zinc | 160 ppm | | | 1600 ppm |
| Diarrhea before feeding | Piglets had constant diarrhea, mental depression, loss of appetite and weight loss. | | | |
| Diarrhea after feeding | There were lumpy feces, basically cured diarrhea, the increased appetite and increased weight in piglets. | | | |
| Average daily feed intake (g/ per pig) | 253.5 | 252.1 | 254.5 | 252.5 |
| Average weight gain (g/ per pig) | 209.5 | 204.9 | 212.0 | 206.9 |
| Feed meat ratio (g/g) | 1.21:1 | 1.23:1 | 1.20:1 | 1.22:1 |
| Death rate % | 0 | 0 | 0 | 0 |
| Residual zinc in 50 g feces | 0 | 0 | 0 | 850 ppm |

It can be seen from Examples 1-3 and Table 4 that, the diarrhea of piglets is significantly improved after feeding piglets with the single atomic zinc additive of the present applicant. Moreover, the average daily feed intake, average daily gain and feed conversion rate of piglets with single atomic zinc additive with zinc content of 160 ppm are the same as or even better than those for zinc oxide. There is no zinc in the feces of piglets after feeding single atomic zinc additive, so that zinc can be repeatedly absorbed by piglets and will not cause environmental pollution. Therefore, it shows that the present application can replace zinc oxide in preventing piglet diarrhea and promoting growth while achieving a more excellent effect.

Table 5 Antimicrobial resistance test of single atomic zinc additive and nano zinc oxide for use in feeds prepared in Examples 1-10 and Comparative Example 1-2

| | Drug-resistant Escherichia coli | Drug-resistant Clostridium welchii | Methicillin-resistant Staphylococcus aureus |
|---|---|---|---|
| Example 1 | 99.2% | 99.1% | 99.2% |
| Example 2 | 99.6% | 99.5% | 99.5% |
| Example 3 | 99.9% | 99.9% | 99.9% |
| Comparative Example 1 | 97.4% | 97.3% | 97.2% |
| Comparative Example 2 | 99.9% | 99.9% | 99.9% |
| Example 4 | 96.5% | 96.4% | 96.5% |
| Example 5 | 98.7% | 98.8% | 98.7% |
| Example 6 | 99.9% | 99.9% | 99.9% |
| Example 7 | 98.9% | 99.0% | 98.9% |
| Example 8 | 98.5% | 98.5% | 98.6% |
| Example 9 | 98.1% | 98.2% | 98.1% |
| Example 10 | 97.8% | 97.8% | 97.7% |
| Nano zinc oxide for use in feeds | 30.8% | 27.5% | 31.1% |

It can be seen from Examples 1-3, Comparative Example 1 and Table 5 that, when the addition amount of single atomic zinc additive is 2000 ppm, the killing rates of single atomic zinc additive to drug-resistant Escherichia coli, drug-resistant Clostridium welchii,

Methicillin-resistant Staphylococcus aureus are better than 2000 ppm zinc oxide. The results show that single atomic zinc additive has better ability of kill piglet diarrhea bacteria than zinc oxide for feeds, without producing drug resistance. When the mass ratio of active metal zinc to nano silica carrier is 1: (100-200), it has excellent ability of killing piglet diarrhea bacteria, without producing drug resistance. In addition, the single atomic zinc additive prepared in Example 3 achieves a killing rate of 99.9%, indicating that, when the mass ratio of active metal zinc to nano silica carrier is 1:100, it has the best effect of killing piglet diarrhea bacteria.

It can be seen from Example 3, Comparative Example 2, Example 6 and Table 5 that, the single atomic zinc additives prepared therein all achieve a killing rate 99.9%, but the raw materials used in Example 3 are less than those in Comparative Example 2 and Example 6. Therefore, Example 3 has better economic benefits.

The above are the preferred embodiments of the present application, which are not intended to limit the protection scope of the present application.

## Claims

1. A single atomic zinc additive for use in feeds, **characterized in that**, the single atomic zinc additive comprises a carrier and active metal zinc, wherein the active metal zinc is distributed on a surface of the carrier in single atomic form, without active metal zinc contained inside the carriers; wherein the carrier is nano silica, and a mass ratio of metal zinc to nano silica is 1:(100-200).

2. A preparation method of the single atomic zinc additive for use in feeds according to claim 1, **characterized in that**, the preparation method comprises the following steps:

   S1: preparation of a nano silica carrier;
   S2: preparation of a single atomic precursor of active metal zinc;
   S3: preparation of single atomic zinc additive precursor: adding the carrier obtained in S1 to a mixed solution obtained in S2 at a speed of 30-60 g/min, performing ultrasonic treatment for 0.2-1 h, stirring at 450-550 rpm for 10-20 h, washing with water to neutral, filtering, drying and grinding to 500 nm to obtain the single atomic zinc additive precursor powder; and
   S4: activation of the single atomic zinc additive precursor powder obtained in S3 at a high temperature to obtain a final product.

3. The preparation method of the single atomic zinc additive for use in feeds according to claim 2, **characterized in that**, a preparation method of the nano silica carrier in S1 comprises the following steps:

   S11: preparing a sodium silicate solution;
   S12: stirring an aqueous ammonium chloride solution under 400-600 rpm at 35-45 °C, and dripping 0.3-0.5 mol/L the sodium silicate solution into the aqueous ammonium chloride solution by 5-15 μL/s until pH is 8.5;
   S13: stirring a mixed solution obtained in S12 under 400-600 rpm for 0.5-1.5 h at 35-45 °C to obtain a precipitate, and centrifugally washing the precipitate with a washing solution; and
   S14: drying the precipitate obtained in S13 at 90-110 °C, and calcining at 480-520 °C for 0.5-1.5 h to obtain the nano silica carrier.

4. The preparation method of the single atomic zinc additive for use in feeds according to claim 3, **characterized in that**, the sodium silicate solution in S11 is prepared by the following steps: preparing an aqueous ethanol solution by a volume ratio of ethanol to water of 1: (7-9); adding sodium silicate solution to the aqueous ethanol solution to obtain a mixed solution of 0.3-0.5 mol/L, and adding cationic surfactant cetyltrimethylammonium bromide to the mixed solution to obtain the sodium silicate solution.

5. The preparation method of the single atomic zinc additive for use in feeds according to claim 4, **characterized in that**, 0.3-0.7 g cationic surfactant cetyltrimethylammonium bromide is added per 100 mL of the sodium silicate solution.

6. The preparation method of the single atomic zinc additive for use in feeds according to claim 5, **characterized in that**, the washing solution in S13 is obtained by mixing a cationic surfactant cetyltrimethylammonium bromide with an aqueous ethanol solution, wherein a volume ratio of ethanol to water in the aqueous ethanol solution is 1: (7-9), and 0.8-1.2 mol of cationic surfactant cetyltrimethylammonium bromide is contained per 100 mL of the aqueous ethanol solution.

7.  The preparation method of the single atomic zinc additive for use in feeds according to claim 2, **characterized in that**, in S2, the single atomic precursor of active metal zinc is prepared by the following steps: adding the sodium carbonate solution to zinc nitrate solution by 5-15 μL/s, stirring under 400-600 rpm for 2-4 h, heating to 55-70 °C within 20-40 min, stirring at the same speed for another 2-4 h, and cooling to room temperature to obtain the single atomic precursor of active metal zinc.

8.  The preparation method of the single atomic zinc additive for use in feeds according to claim 7, **characterized in that**, the aqueous sodium carbonate solution has a mass concentration of 5%, the zinc nitrate solution has a concentration of 100-200 g/L, and a volume ratio of the aqueous sodium carbonate solution to the aqueous zinc nitrate solution is (20-40): 100.

9.  The preparation method of the single atomic zinc additive for use in feeds according to claim 4, **characterized in that**, the activation at a high temperature in S4 comprises: activating the single atomic zinc additive precursor powder obtained in step S3 at a high temperature of 670-740 °C for 1.5-2.5 h in 5% hydrogen-argon mixture atmosphere, cooling, and grinding to 500 nm to obtain the single atomic zinc additive.

**Patentansprüche**

1.  Einatom-Zinkzusatz für Futtermitteln, **dadurch gekennzeichnet, dass** der Einatom-Zinkzusatz einen Träger und ein aktives metallisches Zink umfasst, wobei das aktive metallische Zink in Form eines Einatoms auf der Oberfläche des Trägers verteilt ist und das Innere des Trägers kein aktives metallisches Zink enthält; wobei der Träger ist Nano-Siliciumdioxid und das Massenverhältnis von metallischem Zink zu Nano-Siliciumdioxid beträgt 1: (100-200).

2.  Verfahren zur Herstellung eines Einatom-Zinkzusatzes für Futtermittel nach Anspruch 1, **dadurch gekennzeichnet, dass** das Herstellungsverfahren die folgenden Schritte umfasst:

    S1: Herstellung von Nano-Siliciumdioxid-Trägern;
    S2: Herstellung von einatomaren Vorläufern des aktiven metallischen Zink;
    S3: Herstellung eines Einatom-Zinkzusatz-Vorläufers: Der in S1 erhaltene Träger wird zu der in S2 erhaltenen Mischlösung mit einer Geschwindigkeit von 30-60 g/min zugegeben, 0,2-1 h Ultraschallbehandlung, 10-20 h, bei 450-550 U/min gerührt, mit Wasser neutral gewaschen, filtriert, getrocknet und auf 500 nm gemahlen, um ein Einatom-Zinkzusatz-Vorläuferpulver zu erhalten; und
    S4: Aktivierung des in S3 erhaltenen Einatom-Zinkzusatz-Vorläuferpulvers bei erhöhter Temperatur zum Endprodukt.

3.  Verfahren zur Herstellung eines Einatom-Zinkzusatzes für Futtermittel nach Anspruch 2, **dadurch gekennzeichnet, dass** das Verfahren zur Herstellung von Nano-Siliciumdioxid-Trägern in S1 die folgenden Schritte umfasst:

    S11: Herstellung einer Natriumsilikatlösung;
    S12: Rühren einer wässrigen Ammoniumchloridlösung bei 35-45 °C mit 400-600 U/min und Eintropfen von 0,3-0,5 mol/L Natriumsilikatlösung in die wässrige Ammoniumchloridlösung mit 5-15 μl/s, bis der pH 8,5 beträgt;
    S13: Rühren der in Schritt S12 erhaltenen gemischten Lösung bei 400-600 U/min bei 0,5-1,5 h bei 35-45°C, um einen Niederschlag zu erhalten, der mit einer Waschflüssigkeit zentrifugiert gewaschen wird; und
    S14: Trocknen des in S13 erhaltenen Niederschlags bei 90-110 °C und Kalzinieren bei 480-520°C für 0,5-1,5 h, um den Nano-Siliciumdioxid-Träger zu erhalten.

4.  Verfahren zur Herstellung eines Einatom-Zinkzusatzes für Futtermittel nach Anspruch 3, **dadurch gekennzeichnet, dass** die Natriumsilikatlösung in S11 durch die folgenden Schritte hergestellt wird: eine wässrige Ethanollösung mit einem Volumenverhältnis von Ethanol zu Wasser von 1: (7-9); zu der wäßrigen Ethanollösung wird eine Natriumsilikatlösung zugegeben, um eine Mischlösung von 0,3-0,5 mol/L zu erhalten, und zu der Mischlösung wird ein kationisches oberflächenaktives Cetyltrimethylammoniumbromid zugegeben, um eine Natriumsilikatlösung zu erhalten.

5.  Verfahren zur Herstellung eines Einatom-Zinkzusatzes für Futtermittel nach Anspruch 4, **dadurch gekennzeichnet,**

**dass** 0,3-0,7 g des kationischen oberflächenaktiven Cetyltrimethylammoniumbromid pro 100 mL Natriumsilikatlösung zugegeben werden.

6. Verfahren zur Herstellung eines Einatom-Zinkzusatzes für Futtermittel nach Anspruch 5, **dadurch gekennzeichnet, dass** die Waschflüssigkeit in S13 durch Mischen des kationischen oberflächenaktiven Cetyltrimethylammoniumbromid mit einer wäßrigen Ethanollösung hergestellt wird, wobei das Volumenverhältnis von Ethanol zu Wasser 1:(7-9) in der wässrigen Ethanollösung beträgt und 0,8-1,2 mol des kationischen oberflächenaktiven Cetyltrimethylammoniumbromid pro 100 mL der wäßrigen Ethanollösung enthält.

7. Verfahren zur Herstellung eines Einatom-Zinkzusatzes für Futtermittel nach Anspruch 2, **dadurch gekennzeichnet, dass** in S2 ein einatomarer Vorläufer des aktiven Zinkmetalls durch die folgenden Schritte hergestellt wird: eine Natriumcarbonatlösung zu einer Zinknitratlösung mit einer Geschwindigkeit von 5-15 $\mu$l/s hinzufügt, 2-4 h bei 400-600 U/min gerührt, auf 55-70°C innerhalb von 20-40 min erwärmt, weitere 2-4 h bei gleicher Geschwindigkeit gerührt und auf Raumtemperatur abkühlt, um den einatomaren Vorläufer des aktiven Zinkmetalls zu erhalten.

8. Verfahren zur Herstellung eines Einatom-Zinkzusatzes für Futtermittel nach Anspruch 7, **dadurch gekennzeichnet, dass** die Massenkonzentration der wässrigen Natriumcarbonatlösung 5% beträgt, die Konzentration der Zinknitratlösung 100-200 g/L beträgt und das Volumenverhältnis der wässrigen Natriumcarbonatlösung zu der wässrigen Zinknitratlösung (20-40): 100 beträgt.

9. Verfahren zur Herstellung eines Einatom-Zinkzusatzes für Futtermittel nach Anspruch 4, **dadurch gekennzeichnet, dass** die Hochtemperaturaktivierung in S4 umfasst: Aktivieren des in Schritt S3 erhaltenen einatomaren Zinkzusatz-Vorläuferpulvers bei einer hohen Temperatur von 670-740°C für 1,5-2,5 h in einer Atmosphäre aus einem 5 %igen Wasserstoff-Argon-Gemisch, Abkühlen und Mahlen auf 500 nm, um das Einatom-Zinkzusatz zu erhalten.

## Revendications

1. Additif atomique unique de zinc destiné à être utilisé dans des aliments, **caractérisé en ce que** l'additif atomique unique de zinc comprend un support et du zinc métallique actif, dans lequel le zinc métallique actif est distribué sur une surface du support sous une forme atomique unique, sans zinc métallique actif contenu à l'intérieur des supports ; dans lequel le support est de la nano-silice, et un rapport massique du zinc métallique par rapport à la nano-silice est de 1:(100-200).

2. Procédé de préparation de l'additif atomique unique de zinc destiné à être utilisé dans des aliments selon la revendication 1, **caractérisé en ce que** le procédé de préparation comprend les étapes suivantes :

    S1 : préparation d'un support de nano-silice ;
    S2 : préparation d'un précurseur atomique unique de zinc métallique actif ;
    S3 : préparation d'un précurseur d'additif atomique unique de zinc : ajout du support obtenu en S1 à une solution mixte obtenue en S2 à une vitesse de 30-60 g/min, réalisation d'un traitement par ultrasons pendant 0,2-1 h, agitation à 450-550 tr/min pendant 10-20 h, lavage avec de l'eau à neutre, filtrage, séchage et broyage à 500 nm pour obtenir la poudre de précurseur d'additif atomique unique de zinc ; et
    S4 : activation de la poudre de précurseur d'additif atomique unique de zinc obtenue en S3 à une température élevée pour obtenir un produit final.

3. Procédé de préparation de l'additif atomique unique de zinc destiné à être utilisé dans des aliments selon la revendication 2, **caractérisé en ce qu'**un procédé de préparation du support de nano-silice en S1 comprend les étapes suivantes :

    S11 : préparation d'une solution de silicate de sodium ;
    S12 : agitation d'une solution aqueuse de chlorure d'ammonium sous 400-600 tr/min à 35-45°C, et égouttement à 0,3-0,5 mol/L de la solution de silicate de sodium dans la solution aqueuse de chlorure d'ammonium à 5-15 $\mu$L/s jusqu'à pH 8,5 ;
    S13 : agitation d'une solution mixte obtenue en S12 sous 400-600 tr/min pendant 0,5-1,5 h à 35-45°C pour obtenir un précipité, et lavage centrifuge du précipité avec une solution de lavage ; et
    S14 : séchage du précipité obtenu en S13 à 90-110°C, et calcination à 480-520°C pendant 0,5-1,5 h pour obtenir le support de nano-silice.

**4.** Procédé de préparation de l'additif atomique unique de zinc destiné à être utilisé dans des aliments selon la revendication 3, **caractérisé en ce que** la solution de silicate de sodium en S11 est préparée par les étapes suivantes : préparation d'une solution aqueuse d'éthanol par un rapport volumique de l'éthanol par rapport à l'eau de 1:(7-9) ; ajout d'une solution de silicate de sodium à la solution aqueuse d'éthanol pour obtenir une solution mixte de 0,3-0,5 mol/L, et ajout de bromure de cétyltriméthylammonium tensioactif cationique à la solution mélangée pour obtenir la solution de silicate de sodium.

**5.** Procédé de préparation de l'additif atomique unique de zinc destiné à être utilisé dans des aliments selon la revendication 4, **caractérisé en ce que** 0,3-0,7 g de bromure de cétyltriméthylammonium de tensioactif cationique sont ajoutés pour 100 mL de la solution de silicate de sodium.

**6.** Procédé de préparation de l'additif atomique unique de zinc destiné à être utilisé dans des aliments selon la revendication 5, **caractérisé en ce que** la solution de lavage en S13 est obtenue en mélangeant un bromure de cétyltriméthylammonium tensioactif cationique avec une solution aqueuse d'éthanol, dans lequel un rapport volumique de l'éthanol par rapport à l'eau dans la solution aqueuse d'éthanol est de 1:(7-9), et 0,8-1,2 mole de bromure de cétyltriméthylammonium tensioactif cationique sont contenues pour 100 mL de la solution aqueuse d'éthanol.

**7.** Procédé de préparation de l'additif atomique unique de zinc destiné à être utilisé dans des aliments selon la revendication 2, **caractérisé en ce que**, en S2, le précurseur atomique unique de zinc métallique actif est préparé par les étapes suivantes: ajoute de la solution aqueuse de carbonate de sodium à une solution de nitrate de zinc à 5-15 μL/s, agitation sous 400-600 tr/min pendant 2-4 h, chauffage à 55-70°C pendant 20-40 min, agitation à la même vitesse pendant encore 2-4 h, et refroidissement à température ambiante pour obtenir le précurseur atomique unique de zinc métallique actif.

**8.** Procédé de préparation de l'additif atomique unique de zinc destiné à être utilisé dans des aliments selon la revendication 7, **caractérisé en ce que** la solution aqueuse de carbonate de sodium présente une concentration massique de 5%, la solution de nitrate de zinc présente une concentration de 100-200 g/L, et un rapport volumique de la solution aqueuse de carbonate de sodium par rapport à la solution aqueuse de nitrate de zinc est de (20-40): 100.

**9.** Procédé de préparation de l'additif atomique unique de zinc destiné à être utilisé dans des aliments selon la revendication 4, **caractérisé en ce que** l'activation à une température élevée en S4 comprend : activation de la poudre de précurseur d'additif atomique unique de zinc obtenue à l'étape S3 à une température élevée de 670-740°C pendant 1,5-2,5 h sous une atmosphère de mélange hydrogène-argon à 5%, refroidissement et broyage à 500 nm pour obtenir l'additif atomique unique de zinc.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106186042 B
- CN 109349431 A
- WO 2003077674 A1